(19) Europäisches Patentamt / European Patent Office / Office européen des brevets

(11) **EP 3 068 812 B1**

(12) **EUROPEAN PATENT SPECIFICATION**

(45) Date of publication and mention
of the grant of the patent:
**10.01.2018 Bulletin 2018/02**

(51) Int Cl.:
***C08F 220/18*** *(2006.01)*   ***A61L 27/26*** *(2006.01)*
***C08F 2/46*** *(2006.01)*

(21) Application number: **14827171.1**

(22) Date of filing: **13.11.2014**

(86) International application number:
**PCT/EP2014/003038**

(87) International publication number:
**WO 2015/070981 (21.05.2015 Gazette 2015/20)**

(54) **PROCESS FOR PRODUCING A HYDROPHOBIC ACRYLIC POLYMER MATERIAL FOR AN OPHTHALMIC DEVICE**

VERFAHREN ZUR HERSTELLUNG EINES HYDROPHOBEN ACRYLPOLYMERMATERIALS FÜR EINE OPHTHALMISCHE VORRICHTUNG

PROCÉDÉ DE PRODUCTION D'UN MATÉRIAU POLYMÈRE ACRYLIQUE HYDROPHOBE POUR DISPOSITIF OPHTALMIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.11.2013 FR 1361067**

(43) Date of publication of application:
**21.09.2016 Bulletin 2016/38**

(73) Proprietor: **O & O MDC Limited
Lewes, East Sussex BN7 2NZ (GB)**

(72) Inventor: **MEUNIER, Patrick
00044 FRACASTI (RM) (IT)**

(74) Representative: **Schmidt, Martin Peter
IXAS Conseil
15, rue Emile Zola
69002 Lyon (FR)**

(56) References cited:
**DE-A1- 3 203 655   US-A- 5 290 892**

**Description**

**Technical field of the invention**

The present invention relates to a new process for producing a polymer material suitable for producing ophthalmic devices, and more specifically for producing implantable devices such as intraocular lenses.

**Prior art**

Intraocular lenses are ophthalmological implants or prostheses that are useful for replacing the natural crystalline lens of the eye, which is opacified by the cataract. The replacement of the crystalline lens requires a surgical intervention, in which the surgeon extracts the deficient natural crystalline lens of the patient, replacing it with an intraocular lens. The insertion of the lens into the eye is generally performed by means of a syringe-type injection system.

Intraocular lenses can be in the form of a rigid implant or a flexible implant. Flexible implants have the advantage of being foldable and therefore injectable through a small incision, and therefore easier to position and stabilize in the capsular bag (the organ containing the crystalline lens) than rigid implants. Rigid implants consist essentially of polymethyl methacrylate (PMMA). The materials used for flexible implants are primarily acrylic compound or silicone polymer derivatives.

Flexible implants typically consist of a central optical portion forming the lens and a haptic portion including two or more haptics, located at the periphery of the optical element. These haptic elements are intended to stretch the inner sides of the capsular bag and ensure proper centering of the lens in the eye. They can be annular, flat or handle-shaped. The two portions form the intraocular lens.

Polymer materials for intraocular lenses, whether they are flexible or rigid implants, can be classified into two categories: hydrophilic polymer materials and hydrophobic polymer materials.

Hydrophilic polymer materials are the most deformable, and their flexibility is partially dependent upon their capacity to absorb water. Intraocular lenses produced using these hydrophilic polymer materials have the advantage of containing very few residual compounds, limiting the risk of inflammation of the eye. They are also known for their hyper-biocompatibility, i.e. their capacity to not induce a lens rejection reaction. However, a known problem with cataract extraction is opacification of the posterior capsule, also called a "secondary" cataract, caused by the proliferation and migration of crystalline epithelial cells that migrate to the posterior capsule, i.e. behind the posterior surface of the intraocular lens. Inevitably, a subsequent intervention is necessary in order to cut the posterior capsule, often performed by laser, not to mention the complications that can result from this intervention. Another disadvantage is that hydrophilic lenses must be sterilized and preserved in an aqueous medium, which can create a risk of deterioration of the buffered solution over the medium to long term.

On the other hand, many flexible intraocular lenses obtained using hydrophobic polymer materials have been proposed in the prior art, and are described, for example, in documents US 5,290,892 A, WO 2005/047349 A1, WO 2009/138 591 A1 or WO 2012/106 118 A2. These polymer materials are obtained by conventional polymerization processes and reactions, well known to a person skilled in the art. One of the advantages of hydrophobic lenses is that the posterior capsule of the capsular bag adheres to the hydrophobic polymer material and therefore reduces or prevents the secondary cataract phenomenon. Another advantage is that the sterilization and preservation of these lenses are in "dry" stage, thereby eliminating the risks of deterioration of the aqueous preservation media.

However, these materials have certain disadvantages. Very often, these materials are difficult to purify because they include many water-insoluble residual products. Moreover, a large majority of these materials have an adverse effect referred to as the glistening effect, which modifies the transparency of the optical portion of the intraocular lenses. This phenomenon involves the appearance of microvacuoles of water at any level of the optic, in particular when the polymer material is immersed in aqueous medium: the microvacuoles become visible by the refractive index difference between the water and the polymer. The size of the microvacuoles may vary and be between 5 and 50 $\mu$m in diameter, or even larger. They are generally formed in the months following the implantation of the lens in the patient's eye.

Certain studies have been conducted in order to determine the clinical factors associated with the appearance and severity of the glistening phenomenon in certain hydrophobic acrylic implants (Isabelle Orignac, Joseph Colin; *"Le phénomène de glistening des implants intraoculaires acryliques hydrophobes Acrysof®. facteurs cliniques associés et impact sur la qualité de vision des patients opérés de cataracte"* [*"Glistening in Acrysof® hydrophobic acrylic intraocular lens implants: associated clinical factors and impact on the quality of vision of patients operated for cataract"*]; University of Bordeaux II, 2008). The explanation for the formation of cavities in the material is not yet clearly demonstrated; nevertheless, it appears to be dependent in part on the process of production of the polymer material manufactured for the production of intraocular lenses, the chemical nature of the monomers and the presence of impurities in the polymer material generated during the polymerisation process.

One objective of the invention is to propose a hydrophobic acrylic polymer material that is more resistant to the appearance of water microvacuoles, in particular in the optical portion of the intraocular lenses implanted in the human eye, and thus making it possible to limit light dispersion phenomena in such lenses due in part to Rayleigh scattering and the Tyndall effect.

Another objective of the invention is to propose a hydrophobic acrylic polymer material having a very low impurity content, in terms of residual monomers and/or oligomers and/or pre-polymers, in the final composition. In general, the polymerization reactions are not complete and the polymer formed often contains a non-negligible quantity of residual monomers, oligomers and pre-polymers. These volatile products form what are called volatile organic compounds, hereinafter referred to as VOC, and are subject, on behalf of users, to concentration limits. These specifications, which may vary according to the products, their uses, the nature of the monomers, and the country concerned, are tending to become more strict, and therefore justify the search for processes enabling better compliance.

Another objective of the invention is to propose a material serving as a raw material for the production of a hydrophobic intraocular lens that is biocompatible, i.e. consisting of materials that are accepted by the body and not releasing compounds that are toxic or aggressive to the tissues of the human eye. In effect, intraocular lenses are subject to many functional constraints, due to their nature as they are intended to be implanted inside the human eye, and must satisfy many criteria in order to be considered suitable for that use. Typically, the biocompatibility of an intraocular lens is determined by numerous biocompatibility tests, in particular concerning cytotoxicity, irritation and sensitization.

In the context of the present invention, the applicant has developed a new process for producing a hydrophobic acrylic polymer material making it possible to achieve the aforementioned objectives and in particular a process for producing polymer materials enabling entirely satisfactory intraocular lenses to be developed.

## Subject-matter of the invention

One object of the present invention is a process for preparing a hydrophobic acrylic polymer material for producing an ophthalmic device, which process includes at least:

a) a step of providing a mixture of at least an aromatic acrylate monomer, an aromatic methacrylate monomer and an alkoxyalkyl methacrylate monomer;
b) a step of degassing the mixture obtained in step a);
c) a step of introducing said mixture into an intraocular lens mould;
d) a step of thermally induced free radical polymerization in the presence of an initiator;
e) a step of polymerization by irradiation with gamma rays, and f) a step of vacuum ventilation after step e) of polymerization by irradiation with gamma rays.

Step e) of polymerization by gamma radiation is advantageously performed at a dose of between 20 and 40 kGray, and preferably between 25 and 35 kGray.

The step of thermally induced free radical polymerization is advantageously performed in two heat phases, the temperature of the first phase being between 40 and 70°C and the temperature of the second phase being between 110 and 125°C, preferably between 117 and 122°C.

The ventilation step f) is advantageously performed at a temperature of between 90 and 110°C, and preferably between 97 and 103°C.

Preferably, the monomer mixture includes at least:

i) between 60 and 65% of a 2-phenylethyl acrylate monomer;
ii) between 25 and 30% of a 2-phenylethyl methacrylate monomer;
iii) between 3 and 8% of an alkoxyalkyl methacrylate monomer;
iv) between 0.5 and 1 % methyl methacrylate.

More preferably, the alkoxyalkyl methacrylate is 2-ethoxyethyl methacrylate.

Another object is the hydrophobic acrylic polymer material capable of being obtained by the process according to the invention. Advantageously, its glass transition temperature (Tg) is between 4 and 19°C, and preferably between 11 and 13°C.

This material preferably has a residual content of monomers, oligomers and pre-polymers is less than 0.5% by mass, preferably less than 0.3% by mass, more preferably less than 0.2% by mass, and most preferably less than 0.1 % by mass.

Another object is an intraocular lens blank including the polymer material capable of being obtained by the process according to the invention.

Another object is an ophthalmic device including the polymer material capable of being obtained by the process according to the invention. Preferably, the device is an intraocular lens. Preferably, the residual content of monomers, oligomers and pre-polymers is less than 0.5% by mass, preferably less than 0.3% by mass, and more preferably less than 0.1% by mass. Advantageously said device is essentially free of microvacuoles of a size greater than 7 $\mu$m, and preferably essentially free of microvacuoles of a size greater than 6 $\mu$m, and still more preferably essentially free of microvacuoles of a size greater than 5 $\mu$m.

**Detailed description of the figures**

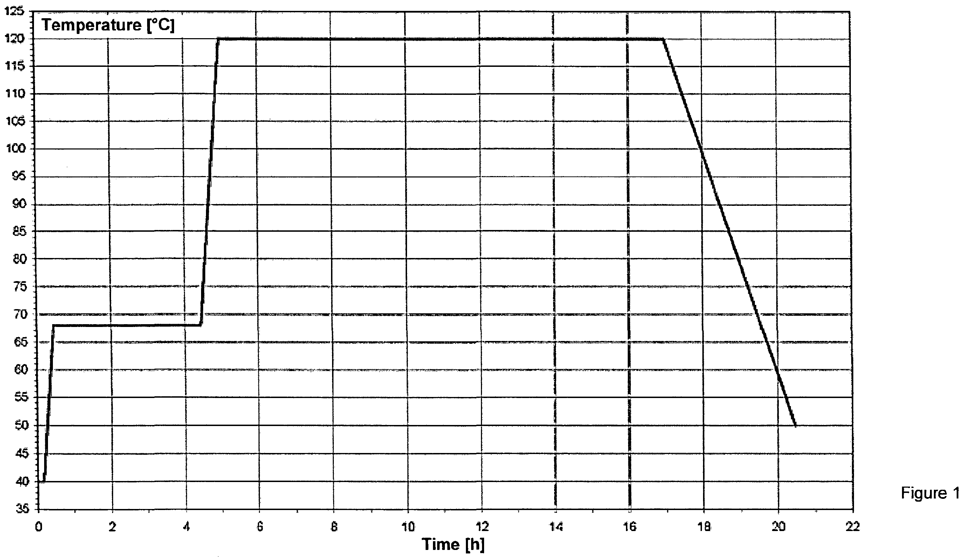

Figure 1 shows the time-dependent temperature profile of the first step of thermally induced polymerization of the monomer mixture of example 2 of the process according to the invention. The x-axis corresponds to the time (in this case, in hours) and the y-axis corresponds to the temperature (in degrees Celsius). A first temperature level of around 65°C was applied for 5 hours, then a second temperature level of 120°C was applied for 12 hours.

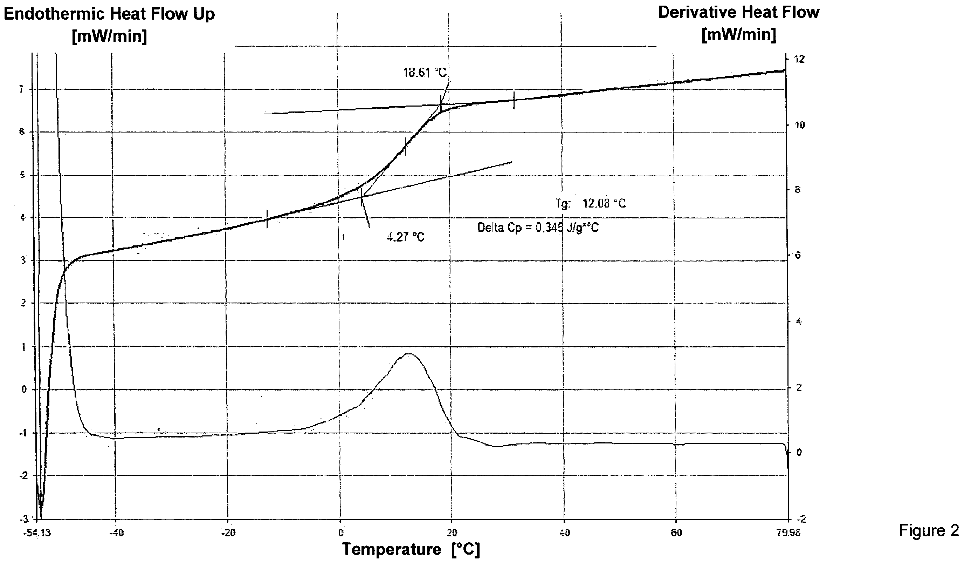

Figure 2 is a DSC thermogram obtained by differential scanning calorimetry of the hydrophobic acrylic polymer material obtained by the process according to the invention (example 2). The top curve shows the DSC of the polymer material of example 2, and the bottom curve shows the temperature-dependent derivative of the heat flux (expressed in mW/min). The glass transition temperature ($T_g$) of the material obtained by the process according to the invention is around 12.1 °C.

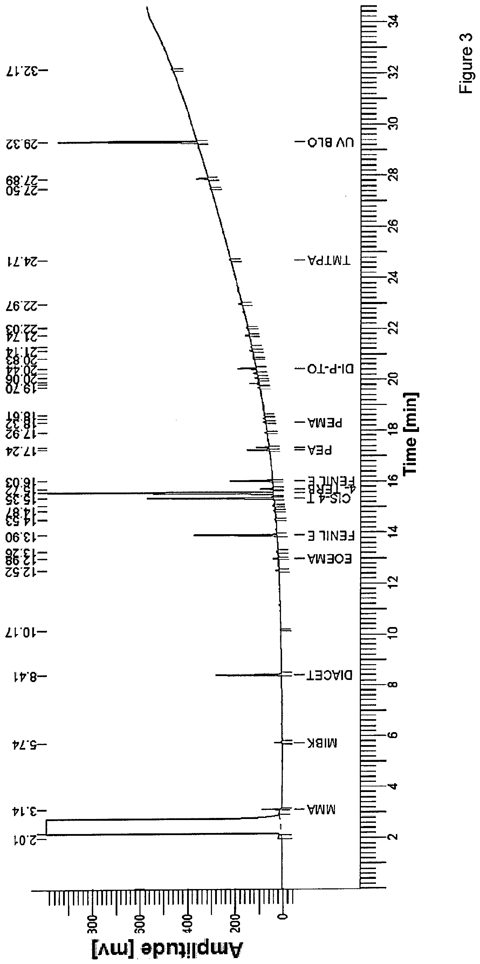

Figure 3 is a chromatogram obtained by gas-phase chromatography (with a flame ionization detector) of a polymer material obtained by a conventional polymerization process known from the prior art (example 1 of the detailed description). The time (in minutes) is shown on the x-axis, and the response (in mV) is shown on the $\gamma$-axis. Ten peaks representing ten compounds can clearly be seen: the first peak (at 3.14 minutes) corresponds to methyl methacrylate, the second peak (at 5.74 minutes) corresponds to methyl isobutyl ketone, the third peak (at 8.41 minutes) corresponds to 4-hydroxy-4-methyl-pentan-2-one, the fourth peak (at 12.98 minutes) corresponds to 2-ethoxyethyl methacrylate, the fifth peak (at 15.35 minutes) corresponds to cis-4-tert-butylcyclohexanol, the sixth peak (at 15.56 minutes) corresponds to trans-4-tert-butylcyclohexanol, the seventh peak (at 17.25 minutes) corresponds to 2-phenylethyl acrylate, the eighth peak (at 18.32 minutes) corresponds to 2-phenylethyl methacrylate, the ninth peak (at 20.44 minutes) corresponds to Di-p-tolylamine, and the tenth peak (at 29.32 minutes) corresponds to the UV blocker compound (in this case Tinuvin®326).

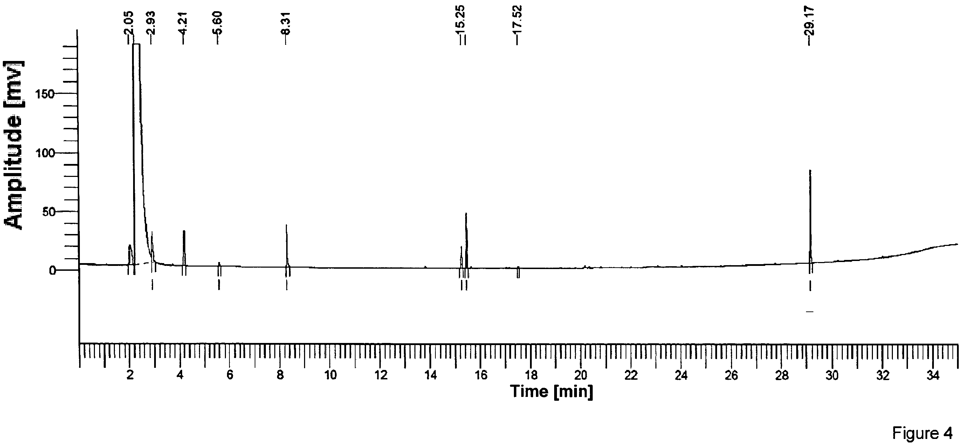

Figure 4 is a chromatogram obtained by gas-phase chromatography (with a flame ionization detector) of the same polymer material obtained by the same conventional polymerisation process as in figure 3, but after multiple vacuum degassing phases (example 1 of the detailed description). The time (in minutes) is shown on the x-axis, and the response (in mV) is shown on the $\gamma$-axis. Six peaks representing six compounds can clearly be seen: the first peak (at 2.94 minutes) corresponds to methyl methacrylate, the second peak (at 5.60 minutes) corresponds to methyl isobutyl ketone, the third peak (at 8.30 minutes) corresponds to 4-hydroxy-4-methyl-pentan-2-one, the fourth peak (at 15.25 minutes) corresponds to cis-4-tert-butylcyclohexanol, the fifth peak (at 15.45 minutes) corresponds to trans-4-tert-butylcyclohexanol, and the sixth peak (at 29.17 min) corresponds to the UV blocker compound (in this case Tinuvin®326).

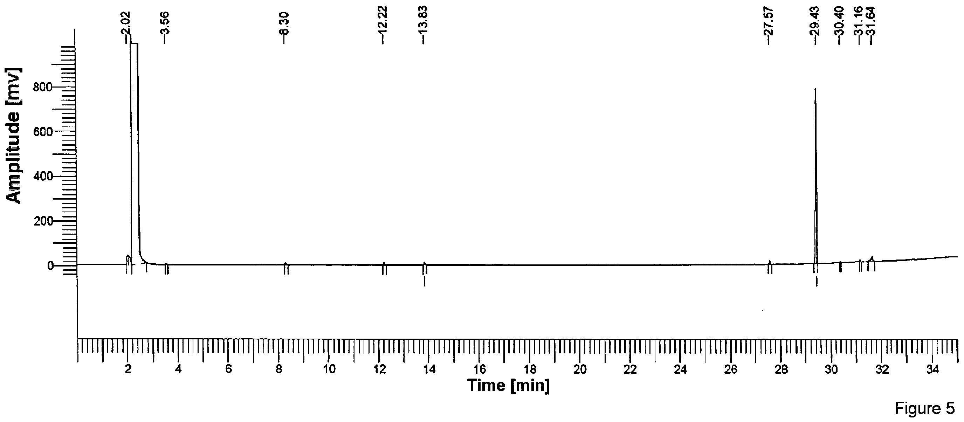

Figure 5 is a chromatogram obtained by gas-phase chromatography (with a flame ionization detector) of a polymer material obtained by the process according to the invention (example 2 of the detailed description). Only two peaks can clearly be seen: the first peak (at 13.83 minutes) corresponds to 2-phenylethanol, and the second peak (at 29.43 minutes) corresponds to the UV blocker compound (in this case Tinuvin®326).

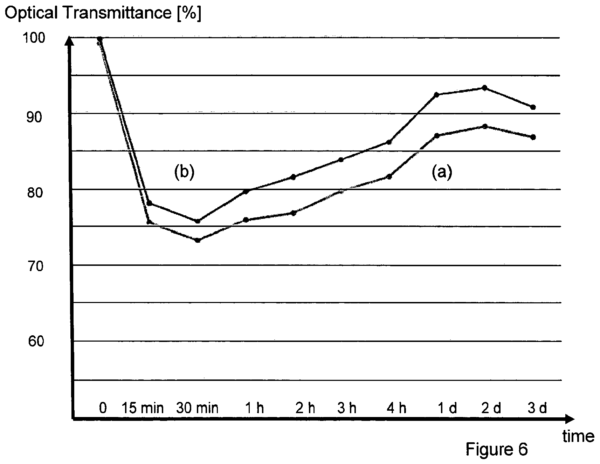

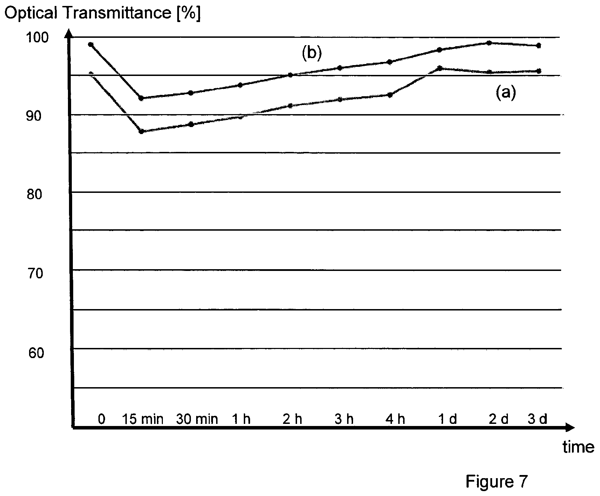

Figures 6 and 7 show the recovery of the optical transmittance at 450 nm (curve (a)) and 800 cm (curve (b)) after artificial glistening of two intraocular lenses obtained from the polymer material obtained by the prior art process described in Example 1 (figure 6) and by the process according to the invention described in Example 2 (figure 7). Glistening was triggered by heating the intraocular lenses in water at 42°C for 72°C, followed by quenching in water at 20°C in which the lenses were maintained for three days for carrying out the reported transmission measurements. The horizontal axis of the figures shows data points on a timeline, the spacing between two adjacent data points being arbitrary. The vertical axis shows the optical transmittance at the given wavelength in percent

**Detailed description of the invention**

**a) Description**

The polymer materials according to the invention are compatible with the human eye and are optically clear (transparent) and suitable for use as a material for an implantable ocular device. The objectives of the invention are achieved with a hydrophobic acrylic polymer material obtained by a specific process and optimized as described in greater detail below.

According to a first aspect, the present invention concerns a process for preparing a hydrophobic acrylic polymer material for an ophthalmic device, which process includes, in the following order, at least:

a) a step of providing a mixture of at least an aromatic acrylate monomer, an aromatic methacrylate monomer and an alkoxyalkyl methacrylate monomer;

b) a step of degassing said mixture;

c) a step of introducing said mixture into an intraocular lens mould;

d) a step of thermally induced free radical polymerization in the presence of an initiator;

e) a step of polymerization by gamma radiation;

f) a step of vacuum ventilation.

Preferably, the monomer mixture according to the invention includes at least an aromatic acrylate monomer, an aromatic methacrylate monomer, an alkoxyalkyl methacrylate monomer and an alkyl methacrylate monomer. More specifically, the initial monomer mixture includes at least 2-phenylethyl methacrylate (also referred to here as PEMA), 2-phenylethyl acrylate (also referred to here as PEA), 2-ethoxyethyl methacrylate (also referred to here as EOEMA) and methyl methacrylate (also referred to here as MMA).

According to the process, the initial acrylate monomer mixture, before polymerization, includes at least:

i) between 60 and 65% by mass of a 2-phenylethyl acrylate monomer (PEA), and more preferably between 62 and 64% by mass;

ii) between 25 and 30% by mass of a 2-phenylethyl methacrylate monomer (PEMA), and more preferably between 26 and 28% by mass;

iii) between 3 and 8% by mass of 2-ethoxyethhyl methacrylate monomer (EOEMA);

iv) between 0.5 and 1% of a methyl methacrylate monomer (MMA).

The polymer materials produced using acrylate monomers tend to have a lower glass transition temperature (Tg) and to be more flexible than the polymer materials produced using methacrylate-type monomers. Consequently, the initial monomer mixture advantageously includes a higher percentage by mass of acrylate-type monomers than methacrylate-type monomers.

Aromatic monomers, whether they are of the acrylate or methacrylate type, make it possible to increase the refractive index of the polymer material resulting from the process of the invention. However, these compounds tend to increase the rigidity of the polymer material. That is why the initial monomer mixture advantageously includes an alkoxyalkyl methacrylate monomer. The applicant also discovered, surprisingly, that providing a clearly defined quantity of alkoxyalkyl methacrylate in the initial monomer mixture makes it possible to reduce the glistening phenomenon. Advantageously, the alkoxyalkyl methacrylate is 2-ethoxyethyl methacrylate. These monomers make it possible to reinforce the elasticity of the intraocular lens.

The use of methyl methacrylate, even in a very small mass quantity in the monomer mixture (typically <1% by mass of the total composition of the monomer mixture), makes it possible to optimise the mechanical properties of the resulting material, in particular by increasing the elasticity and reducing the adhesiveness, at temperatures below 25°C, of the resulting material.

The crosslinking agent used for the thermally induced polymerization reaction is preferably a compound having at least two polymerisable groups with ethylene unsaturation, which enables a three-dimensional network to be formed. Ethylene glycol dimethacrylate (EGDMA) and trimethylolpropane triacrylate (TMPTA) are preferred. The initial mixture can also include a mixture of multiple crosslinking agents, for example a mixture of ethylene glycol dimethacrylate and trimethylolpropane triacrylate. Preferably, the total mass quantity of crosslinking agent(s) introduced into the initial acrylate monomer mixture is between 3 and 5% of the total mass of the initial mixture, and preferably between 4 and 5%.

In addition to the monomers, the initial mixture can include a certain number of additional compounds of different types. For example, it can include a polymerization initiator. The polymerisation initiators typically used are 2,2'-azo-bis(isobutyronitrile), also called AIBN, or bis(tert-butylcyclohexyl) peroxydicarbonate. AIBN is particularly preferred in view of its low toxicity, as well as that of its degradation products. In general, the polymerization initiator is involved in a very small quantity and is removed at the end of the preparation process according to the invention. Preferably, the mass quantity of initiator does not exceed 0.2% by mass, and preferably 0.15% by mass, of the total mass of the initial mixture.

Advantageously, the initial mixture can include an ultraviolet stabilizer for preventing the degradation caused by exposure to ultraviolet radiation. Tinuvin®326 or UV 416 are preferred. Preferably, the mass quantity of ultraviolet stabilizer introduced into the monomer composition is between 0.3 and 0.6% of the total mass of the initial mixture, and preferably between 0.4 and 0.5% by mass.

In a particular embodiment, after the step of providing the initial mixture, it is possible to bubble an inert gas, for example argon, in the mixture under stirring, for example for 15 minutes, and the mixture is subjected to a dynamic vacuum, for example for 30 minutes. The inert gas/vacuum bubbling cycle is repeated preferably at least 3 times.

In another embodiment, the degassing step is performed by stirring the mixture for several minutes (around 30 minutes) under reduced pressure.

Step d) of thermally induced polymerization is performed in a first phase at a temperature of between 40°C and 70°C, preferably around 65°C. Then, in a second phase, the polymerization is performed at a temperature of between 110 and 125°C, preferably between 110 and 122°C. The thermally induced polymerization reaction time is between 15 and 24 hours, and preferably between 20 and 21 hours.

Step e) of polymerization irradiation with gamma rays is preferably performed at a dose of between 20 and 40 kGray, and more preferably between 25 and 35 kGray, taking into consideration the positioning of the reaction mixture with respect to the source. The applicant discovered, surprisingly, that this step makes it possible to considerably reduce the impurity content (monomers, oligomers and/or pre-polymers) in the polymer material, while increasing its resistance to glistening (cf. examples 1 and 2 below): this is the radiodegradation phenomenon. In addition, this step makes it possible to terminate the formation of intermolecular and intramolecular bonds: this is the radiocrosslinking phenomenon. The reaction time is very short and is typically several hundredths of seconds. Typically the irradiation with gamma rays is performed using a Cobalt 60 source.

According to a preferred embodiment, the two polymerization steps, by heat and by irradiation with gamma rays, are performed while the reaction mixture remains in the intraocular lens mould so that, after these two steps, the polymer material is recovered in the form of a pre-moulded intraocular lens.

Finally, a last essential step according to the process of the invention consists of ventilation of the polymer material obtained after the polymerization reactions. This step makes it possible, on the one hand, to remove the residual impurities that may remain in the polymer material, and, on the other hand, to restore the optical clarity of the polymer material. In effect, during the step of irradiation with gamma radiation, the polymer material obtained may lose its light transmission quality, resulting physically in a slight yellowing of the material. This phenomenon is explained by the formation of free radicals by radiodegradation capable of forming temporarily or permanently coloured spaces. The ventilation step according to the invention makes it possible to eliminate the aforementioned disadvantages. This step is performed on at least one of the two faces of the polymer obtained. For this, the polymer material obtained after the two polymerization reactions is placed under a reduced pressure of between 500 and 1000 mbars and at a temperature of between 90 and 110°C, preferably between 97 and 103°C. This treatment typically lasts between 12 and 48 hours according to the temperature chosen. Typically, the treatment time is 24 hours. This step makes it possible to remove the rest of the volatile compounds present at the surface of the polymer material obtained and makes it possible to further reduce the monomer, oligomer or pre-polymer content resulting in particular from the synthesis of each of the monomers used and the irradiation of the reaction mixture by gamma radiation. According to the process of the invention, the polymer materials obtained include a residual compound content of less than 1% by mass, preferably less than 0.5% by mass, and more preferably less than 0.1% by mass. This very low residual compound content eliminates numerous steps of treatment of the polymer material (in particular surface treatments) during the machining thereof for producing the ophthalmic device.

According to another aspect of the process, an additional blue light filter is added to the initial mixture in order to filter the visible light below 400 nm and reduce the transmission of high-energy blue light between 400 and 500 nm (less than 50% transmission for wavelengths below 450 nm). This filter restores the natural protection of the eye against the deleterious effects of blue light for the retina. Typically, N-2-[3(2-methyl phenyl azol)-4-hydroxyphenyl] ethyl methacrylamide or 4-[(E)-phenyldiazenyl]phenyl-2-methacrylate are used as a chromophore.

The hydrophobic acrylic polymer material preferably has a refractive index in the hydrated state of at least around 1.50 and more preferably at least around 1.52 as measured by the Peltier-effect refractometer at 546.1 nm and 589.3 nm and at 20°C and 35°C.

The mass quantities of the monomers included in the polymer materials obtained according to the process of the present invention are chosen so that the polymer material has a glass transition temperature (Tg) that does not exceed 37°C, which corresponds to the normal human body temperature. More specifically, it is preferable to use a polymer material having a glass transition temperature of between 4°C and 19°C. In effect, the placement of an intraocular lens in the patient's eye is normally performed in an operating room in which the temperature is around 19°C. Thus, during the intervention, the handling of the intraocular lens will be much easier because, as the glass transition temperature of the polymer material is below the temperature of the operating room, it will be more flexible and therefore easier to deform and inject into the patient's eye. In addition, the production of a polymer material having such a glass transition temperature, i.e. between 4°C and 19°C, makes it possible to easily machine it in order to obtain a conforming intraocular lens, for example by lathing-milling, and therefore without having to use much more complex machining methods, such as cryogenic machining.

In addition, for use as flexible intraocular lenses, the polymer materials obtained by the process of the present invention must be strong enough for the resulting intraocular lenses to be capable of being folded and manipulated without the risk of appearance of cracks. Consequently, the polymer materials have an elongation of at least 80%, preferably at least 100%, and preferably between 110 and 200% at 20°C.

The polymer materials obtained by the process according to the invention preferably have a saturation water content of 0.5 to 3% by mass, which is calculated using the following formula:

$$\text{Saturation water content (\%)} = \frac{\text{wet mass - dry mass}}{\text{wet mass}} \times 100$$

(Equation 1)

Finally, the polymer materials obtained by the process according to the invention are more resistant to glistening than the polymer materials known from the prior art (as will be demonstrated in the examples below).

The intraocular lens blank made of the material capable of being obtained by the process according to the invention can then be formed into an intraocular lens according to conventional methods for a person skilled in the art, for example by machining at low temperature, for example with a lath/mill machine, and without having to use cryogenic machining. The machining can be performed at a temperature slightly above the glass transition temperature of the polymer material.

To better describe the invention, an example of a hydrophobic acrylic polymer material according to the invention has been described in detail below. Of course, the invention is not limited to this embodiment, and a person skilled in the art can obviously make modifications without going beyond the scope or the context of the present invention.

**b) Examples**

The invention is illustrated below with two examples, which in no way limit the invention. Examples 1 and 2 relate to the preparation of a hydrophobic acrylic polymer material with the same monomers and the same quantities (expressed in % by mass) of starting monomers. First, we describe the preparation of the hydrophobic acrylic polymer material using a conventional polymerization process well known from the prior art.

For the two examples, the initial mixture includes the following compounds:

i) Monomers

- 2-Phenylethyl acrylate (CAS: 3530-36-7)
- 2-Phenylethyl methacrylate (CAS: 3683-12-3)
- 2-Ethoxyethyl methacrylate (CAS: 2370-63-0)
- Methyl methacrylate (CAS: 80-62-6)

ii) Crosslinking agent(s)

- Ethylene Glycol Dimethacrylate (CAS: 97-90-5)
  and/or
- Trimethylolpropane Triacrylate (CAS: 15625-89-5)

iii) Initiator Compound

- 2,2'-azobis(isobutyronitrile) (CAS: 78-67-1)
  or
  Bis(tert-butylcyclohexyl) peroxydicarbonate (CAS: 15520-11-3)

iv) Ultraviolet blocker

- Tinuvin® 326 (CAS: 3896-11-5)
  or
  2-(4-Benzoyl-3-hydroxyphenoxy) ethyl acrylate (CAS: 16432-81-8), commonly called UV 416

For the two examples, several comparative analyses were performed, concerning in particular the purity of the polymer material (analyzed by gas-phase chromatography) and by a "glistening" test, which will be described in detail below. The different parameters of the polymer materials obtained are listed below:

a) Refractive index: The refractive index was determined according to the standard ASTM D542-2000 using a Peltier-effect refractometer (Schmidt-Haensch).

b) Glass transition temperature: The glass transition temperature was determined by differential scanning calorimetry according to the standard ASTM D3418-03: 2000 using a DSC 400 calorimeter (Perkin Elmer).

c) Hardness: The hardness test (Shore A) was determined using the standard ASTM D2240: 2000 using the Hildebrand Shore A.

d) Saturation water content: The saturation water content is expressed in % by mass and was determined using the formula according to equation 1 (cf. detailed description).

e) Elongation test: The elongation test was performed using a Shimadzu EZ or MARK 10 measurement bench.

f) Residual compound content: The impurities (monomers and/or oligomers and/or pre-polymers) were analyzed by gas-phase chromatography with a flame ionization detector (Instrument used: Clarius 580 of the Perkin Elmer company).

g) Microvacuole size: measured by means of an Olympus BX 51M microscope with bright, dark background lighting with phase contrast.

h) Glistening: As the glistening phenomenon occurs only after the intraocular lens has been implanted in the patient's eye, and after several weeks post-operation, a test was developed to trigger glistening in order to compare the response of different polymer materials. The test was performed as follows: intraocular lenses (produced using a polymer material resulting from the process according to the invention or a conventional process) were placed in an aqueous solution for 72 hours at 42°C, then were suddenly quenched in water at 20°C (to obtain maximum temperature shock). The lenses were analyzed at $T_0$; $t_1$=15 minutes; $t_2$=30 minutes; $t_3$=1 hour; $T_4$=2 hours; $T_5$=3 hours; $T_6$=4 hours; $T_7$=1 day; $T_8$=2 days and $T_9$=3 days after the lens had been kept in water at 20°C.

i) Kinetics of recovery from artificial glistening: In a similar experiment to that reported under point h) above, glistening was triggered by heating the intraocular lenses in water at 42°C for 72°C, followed by quenching in water at 20°C in which the lenses were maintained for three days and removed from time to time for carrying out the reported transmission measurements. During this test the intraocular lenses are submitted to conditions that are much stricter than under use conditions, and this test is considered as a good simulation of the long term behavior of the lenses, knowing that for intraocular lenses a minimum transmittance value in the visible spectrum of 90% or better over their whole lifetime is a usual requirement.

Example 1 (conventional polymerization process)

The following initial mixture was produced at room temperature until a homogeneous and clear liquid mixture was obtained (the percentages are expressed by mass):

- 2-Phenylthyl acrylate (CAS : 3530-36-7): 63.0%
- 2-Phenylethyl methacrylate (CAS: 3683-12-3): 27.2%
- 2-Ethoxyethyl methacrylate (CAS: 2370-63-0): 3.80%
- Methyl methacrylate (CAS: 80-62-6): 0.70%
- Ethylene Glycol Dimethacrylate (CAS: 97-90-5): 4.70%
- Bis(tert-butylcyclohexyl) peroxydicarbonate (CAS: 15520-11-3): 0.10%
- Tinuvin® 326 (CAS: 3896-11-5): 0.50%

The mixture was stirred for around 20 minutes. Then, a degassing step was performed for 15 minutes. The mixture was pre-filtered and was then injected into an intraocular lens mould.

The thermally induced free-radical polymerization step was performed at a temperature of around 45°C for 5 hours, then at 125°C for 12 hours. The polymer material obtained was then placed under vacuum for 48 hours at a temperature of 60°C.

Results

a) Refraction index: 1.523 (546.1 nm at 23°C)
b) Glass transition temperature (Tg): + 11°C
c) Hardness: 62 Shore A
d) Saturation water content: 1.1 % by mass
e) Elongation test: undetermined
f) Residue content: between 0.5 and 1.8 % by mass (cf. figure 4 and figure 3)
g) Microvacuole size: 5 to 17 $\mu$m
h) Glistening: after one hour, the glistening of the lens produced using the polymer material obtained by conventional polymerization remains entirely opaque. After 24 hours, the lens remains opaque, but less intensely so. After 36 hours, the lens is only slightly opaque.

i) Kinetics of recovery from artificial glistening: The curve is shown on figure 6. The glistening effect is strong (the transmittance drops to less than 75%) and recovery is incomplete and hardly exceeds an average value of 85%.

Example 2 (process according to the invention)

The following initial mixture was produced at room temperature until a homogeneous and clear liquid mixture was obtained (the percentages are expressed by mass):

- 2-Phenylethyl acrylate (CAS: 3530-36-7): 63.0%
- 2-Phenylethyl methacrylate (CAS: 3683-12-3): 27.2%
- 2-Ethoxyethyl methacrylate (CAS: 2370-63-0): 3.80%
- Methyl methacrylate (CAS: 80-62-6): 0.70%
- Ethylene Glycol Dimethacrylate (CAS: 97-90-5): 4.70%
- 2,2'-azobis(isobutyronitrile) (CAS: 78-67-1): 0.10%
- Tinuvin® 326 (CAS: 3896-11-5): 0.50%

The mixture was stirred for around one hour. Then, a degassing step was performed for 30 minutes at a pressure of 0.3 bar. The mixture was pre-filtered before being injected into an intraocular lens mould.

The thermally induced free radical polymerization step was performed at a temperature of around 65°C for 5 hours, then at 120°C for 12 hours (cf. figure 1). Then, the step of polymerization by irradiation with gamma radiation was performed at a dose corresponding to 28 kGray. The polymer material obtained was then placed under a 1000 mbarsvacuum for 24 hours at a temperature of 100°C.

Results

a) Refraction index: 1.526 (546.1 nm at 23°C)

b) Glass transition temperature (Tg): + 12.1 °C (figure 2)

c) Hardness: 60 $\pm$ 2 Shore A

d) Saturation water content: < 1% by mass

e) Elongation test: > 200% at 20°C

f) Residue content: < 0.1 % by mass (cf. figure 5)

g) Microvacuole size: 3 to 5 $\mu$m

h) Glistening: after one hour, the glistening of the lens produced using the polymer material according to the invention begins to fade very visibly because a target can be deciphered through the lens. After 24 hours, the glistening of the lens has practically disappeared; only the central area (< 2 mm) of the lens has several microvacuoles, but the overall transparency of the lens has returned. After 36 hours, there is no more glistening on the lens.

i) Kinetics of recovery from artificial glistening: The curve is shown on figure 7. The glistening effect is weak (the transmittance drops to an average value of higher than 85%). Recovery leads to an average value over 90% for the blue ray of the spectrum, and is almost complete (about 98%) for the red ray of the spectrum. This is a very significant improvement over prior art (see example 1) which allows to use this material for the manufacture of intraocular lenses having a long life-time.

The results obtained for the hydrophobic acrylic polymer material produced using the process according to the invention clearly demonstrate the very small quantity of impurities (< 0.1 %) and better resistance to glistening.

Moreover, the glistening tests clearly show a reduction in the size of the microvacuoles, of which the diameter is between 3 and 5 $\mu$m, which has the effect of attenuating or even preventing the dispersion of light in the intraocular lens, and consequently the Rayleigh scattering and the Tyndall effect.

Finally, the reduction of the microvacuole size also enables, in the forced glistening test, quick recovery of the transparency of the polymer material, which is a remarkable effect of the polymer materials obtained by the process according to the invention.

## Claims

1. Process for preparing a hydrophobic acrylic polymer material for the production of an ophthalmic device, which process includes at least:

   a) a step of providing a mixture of at least an aromatic acrylate monomer, an aromatic methacrylate monomer and an alkoxyalkyl methacrylate monomer;
   b) a step of degassing the mixture obtained in step a);
   c) a step of introducing said mixture into an intraocular lens mould;
   d) a step of thermally induced free radical polymerization in the presence of an initiator;
   e) a step of polymerization by irradiation with gamma rays; and
   f) a step of vacuum ventilation after step e) of polymerization by irradiation with gamma rays.

2. Process according to claim 1, **characterized in that** step e) of polymerization by gamma radiation is performed at a dose of between 20 and 40 kGray, and preferably between 25 and 35 kGray.

3. Process according to any one of claims 1 or 2, **characterized in that** the step of thermally induced free radical polymerization is performed in two heat phases, the temperature of the first phase being between 40 and 70°C and the temperature of the second phase being between 110 and 125°C, and preferably between 117 and 122°C.

4. Process according to any one of claims 1 to 3, **characterized in that** the ventilation step f) is performed at a temperature of between 90 and 110°C, and preferably between 97 and 103°C.

5. Process according to any one of claims 1 to 4, **characterized in that** the monomer mixture includes at least:

   i) between 60 and 65% of a 2-phenylethyl acrylate monomer;
   ii) between 25 and 30% of a 2-phenylethyl methacrylate monomer;
   iii) between 3 and 8% of an alkoxyalkyl methacrylate monomer, and preferably 2-ethoxyethyl methacrylate.;
   iv) between 0.5 and 1% methyl methacrylate.

6. Hydrophobic acrylic polymer material capable of being obtained by the process according to any one of claims 1 to 5.

7. Material according to claim 6, **characterized in that** the glass transition temperature (Tg) is between 4 and 19°C, and preferably between 11 and 13°C.

8. Material according to claims 6 or 7, **characterized in that** its residual content of monomers, oligomers and prepolymers is less than 0.5% by mass, preferably less than 0.3% by mass, and more preferably less than 0.1 % by mass.

9. Intraocular lens blank including the polymer material according to any of claims 6 to 8, or the polymer material capable of being obtained by the process according to any one of claims 1 to 5.

10. Ophthalmic device including the polymer material according to any of claims 6 to 8, or the polymer material capable of being obtained by the process according to any one of claims 1 to 5.

11. Ophthalmic device according to claim 10, wherein said device is an intraocular device, and preferably an implantable device.

12. Ophthalmic device according to claim 11, wherein sad device is an intraocular lens.

13. Ophthalmic device according to any of claims 11 or 12, **characterized in that** said device has a residual content of monomers, oligomers and pre-polymers less than 0.5% by mass, preferably less than 0.3% by mass, and more preferably less than 0.1 % by mass.

14. Ophthalmic device according to any of claims 11 to 13, wherein said device is essentially free of microvacuoles of a size greater than 7 $\mu$m, and preferably essentially free of microvacuoles of a size greater than 5 $\mu$m.

**Patentansprüche**

1. Verfahren für die Zubereitung eines hydrophilen Acrylpolymermaterials für die Herstellung einer ophthalmischen Vorrichtung, wobei das Verfahren zumindest Folgendes enthält:

   a) einen Schritt des Bereitstellens einer Mischung aus zumindest einem aromatischen Acrylatpolymer, einem aromatischen Methacrylatmonomer und einem Alkoxyalkylmethacrylatmonomer;
   b) einen Schritt des Entgasens der in Schritt a) erhaltenen Mischung;
   c) einen Schritt des Einführens der Mischung in eine Intraokularlinsenform;
   d) einen Schritt der thermal induzierten Radikalpolymerisation in Anwesenheit eines Initiators;
   e) einen Schritt der Polymerisation durch Bestrahlung mit Gammastrahlen; und
   f) einen Schritt der Vakuumbelüftung nach Schritt e) der Polymerisation durch Bestrahlung mit Gammastrahlen.

2. Verfahren nach Anspruch 1, **dadurch gekennzeichnet, dass** Schritt e) der Polymerisation durch Gammabestrahlung mit einer Dosis von zwischen 20 und 40 kGray und bevorzugt zwischen 25 und 35 kGray durchgeführt wird.

3. Verfahren nach einem der Ansprüche 1 oder 2, **dadurch gekennzeichnet, dass** der Schritt der thermal induzierten Radikalpolymerisation in zwei Hitzephasen durchgeführt wird, wobei die Temperatur der ersten Phase zwischen 40 und 70 °C und die Temperatur der zweiten Phase zwischen 110 und 125 °C, und bevorzugt zwischen 117 und 122 °C beträgt.

4. Verfahren nach einem der Ansprüche 1 bis 3, **dadurch gekennzeichnet, dass** der Belüftungsschritt f) bei einer Temperatur von zwischen 90 und 110 °C und bevorzugt zwischen 97 und 103 °C durchgeführt wird.

5. Verfahren nach einem der Ansprüche 1 bis 4, **dadurch gekennzeichnet, dass** die Monomermischung zumindest Folgendes enthält:

   i) zwischen 60 und 65 % eines 2-Phenylethylacrylatmonomers;
   ii) zwischen 25 und 30 % eines 2-Phenylethylmethacrylatmonomers;
   iii) zwischen 3 und 8 % eines Alkoxyalkylmethacrylatmonomers und bevorzugt 2-Ethoxyethylmethacrylat;
   iv) zwischen 0,5 und 1 % Methylmethacrylat.

6. Hydrophobes Acrylpolymermaterial, das in der Lage ist, durch das Verfahren nach einem der Ansprüche 1 bis 5 erhalten zu werden.

7. Material nach Anspruch 6, **dadurch gekennzeichnet, dass** die Glasübergangstemperatur (Tg) zwischen 4 und 19 °C und bevorzugt zwischen 11 und 13 °C beträgt.

8. Material nach Anspruch 6 oder 7, **dadurch gekennzeichnet, dass** sein Restgehalt an Monomeren, Oligomeren und Präpolymeren weniger als 0,5 Massen-%, bevorzugt weniger als 0,3 Massen-% und noch eher bevorzugt weniger als 0,1 Massen-% beträgt.

9. Intraokularlinsen-Rohling, enthaltend das Polymermaterial nach einem der Ansprüche 6 bis 8 oder das Polymermaterial, das in der Lage ist, durch das Verfahren nach einem der Ansprüche 1 bis 5 erhalten zu werden.

10. Ophthalmische Vorrichtung, enthaltend das Polymermaterial nach einem der Ansprüche 6 bis 8 oder das Polymermaterial, das in der Lage ist, durch das Verfahren nach einem der Ansprüche 1 bis 5 erhalten zu werden.

11. Ophthalmische Vorrichtung nach Anspruch 10, wobei die Vorrichtung eine intraokulare Vorrichtung und bevorzugt eine implantierbare Vorrichtung ist.

12. Ophthalmische Vorrichtung nach Anspruch 11, wobei die Vorrichtung eine Intraokularlinse ist.

13. Ophthalmische Vorrichtung nach einem der Ansprüche 11 oder 12, **dadurch gekennzeichnet, dass** die Vorrichtung einen Restgehalt an Monomeren, Oligomeren und Präpolymeren weniger als 0,5 Massen-%, bevorzugt weniger als 0,3 Massen-% und noch eher bevorzugt weniger als 0,1 Massen-% aufweist.

14. Ophthalmische Vorrichtung nach einem der Ansprüche 11 bis 13, wobei die Vorrichtung im Wesentlichen frei von Mikrovakuolen mit einer Größe von mehr als 7 μm und bevorzugt im Wesentlichen frei von Mikrovakuolen mit einer Größe von mehr als 5 μm ist.

**Revendications**

1. Procédé de préparation d'un matériau polymère acrylique hydrophobe pour la production d'un dispositif ophtalmologique, lequel procédé inclut au moins :

   a) une étape de fourniture d'un mélange d'au moins une monomère d'acrylate aromatique, un monomère de méthacrylate aromatique, et un monomère de méthacrylate d'alcoxyalkyle ;
   b) une étape de dégazage du mélange obtenu à l'étape a) ;
   c) une étape d'introduction dudit mélange dans un moule pour lentille intraoculaire ;
   d) une étape de polymérisation radicalaire par voie thermique en présence d'un initiateur ;
   e) une étape de polymérisation par irradiation aux rayons gamma ; et
   f) une étape de ventilation sous vide après l'étape e) de polymérisation par irradiation aux rayons gamma.

2. Procédé selon la revendication 1, **caractérisé en ce que** l'étape e) de polymérisation par rayonnement gamma est réalisée à une dose comprise entre 20 et 40 kGray, et de préférence entre 25 et 35 kGray.

3. Procédé selon l'une quelconque des revendications 1 ou 2, **caractérisé en ce que** l'étape de polymérisation radicalaire par voie thermique est réalisée en deux phases thermiques, la température de la première phrase étant comprise entre 40 et 70 °C, et la température de la seconde phase étant comprise entre 110 et 125 °C, de préférence entre 117 et 122 °C.

4. Procédé selon l'une quelconque des revendications 1 à 3, **caractérisé en ce que** l'étape de ventilation f) est réalisée à une température comprise entre 90 et 110 °C, et de préférence entre 97 et 103 °C.

5. Procédé selon l'une quelconque des revendications 1 à 4, **caractérisé en ce que** le mélange de monomères inclut au moins :

   i) entre 60 et 65 % d'un monomère d'acrylate de 2-phényléthyle ;
   ii) entre 25 et 30 % d'un monomère méthacrylate de 2-phényléthyle ;
   iii) entre 3 et 8 % d'un monomère de méthacrylate d'alcoxyalkyle, et de préférence de méthacrylate de 2-éthoxyéthyle ;
   iv) entre 0,5 et 1 % de méthacrylate de méthyle.

6. Matériau polymère acrylique hydrophobe pouvant être obtenu par le procédé selon l'une quelconque des revendications 1 à 5.

7. Matériau selon la revendication 6, **caractérisé en ce que** la température de transition vitreuse (Tg) est comprise entre 4 et 19 °C, et de préférence entre 11 et 13 °C.

8. Matériau selon les revendications 6 ou 7, **caractérisé en ce que** la teneur résiduelle en monomères, oligomères et prépolymères est inférieure à 0,5 % en masse, de préférence inférieure à 0,3 % en masse, et de manière davantage préférée inférieure à 0,1 % en masse.

9. Ébauche de lentille intraoculaire incluant le matériau polymère selon l'une quelconque des revendications 6 à 8, ou le matériau polymère pouvant être obtenu par le procédé selon l'une quelconque des revendications 1 à 5.

10. Dispositif ophtalmologique incluant le matériau polymère selon l'une quelconque des revendications 6 à 8, ou le matériau polymère pouvant être obtenu par le procédé selon l'une quelconque des revendications 1 à 5.

11. Dispositif ophtalmologique selon la revendication 10, dans lequel ledit dispositif est un dispositif intraoculaire, et de préférence un dispositif implantable.

12. Dispositif ophtalmologique selon la revendication 11, dans lequel ledit dispositif est une lentille intraoculaire.

**13.** Dispositif ophtalmologique selon l'une quelconque des revendications 11 ou 12, **caractérisé en ce que** ledit dispositif a une teneur résiduelle en monomères, oligomères et prépolymères inférieure à 0,5 % en masse, de préférence inférieure à 0,3 % en masse, et de manière davantage préférée inférieure à 0,1 % en masse.

**14.** Dispositif ophtalmologique selon l'une quelconque des revendications 11 à 13, dans lequel ledit dispositif est sensiblement dépourvu de microvacuoles d'une taille supérieure à 7 $\mu$m, et de préférence sensiblement dépourvu de microvacuoles d'une taille supérieure à 5 $\mu$m.

Figure 1

14

Figure 2

Figure 3

Figure 4

Figure 5

Figure 6

Figure 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5290892 A
- WO 2005047349 A1
- WO 2009138591 A1
- WO 2012106118 A2

### Non-patent literature cited in the description

- *CHEMICAL ABSTRACTS,* 3530-36-7
- *CHEMICAL ABSTRACTS,* 3683-12-3
- *CHEMICAL ABSTRACTS,* 2370-63-0
- *CHEMICAL ABSTRACTS,* 80-62-6
- *CHEMICAL ABSTRACTS,* 97-90-5
- *CHEMICAL ABSTRACTS,* 15625-89-5
- *CHEMICAL ABSTRACTS,* 78-67-1
- *CHEMICAL ABSTRACTS,* 15520-11-3
- *CHEMICAL ABSTRACTS,* 3896-11-5
- *CHEMICAL ABSTRACTS,* 16432-81-8